# EUROPÄISCHE PATENTANMELDUNG

(11) **EP 2 835 070 A1**
(43) Veröffentlichungstag der Anmeldung: **11.02.2015**
(21) Anmeldenummer: 14002348.2
(22) Anmeldetag: 08.07.2014
(51) Int. Cl.: A43B 3/00, A43B 7/02, A43B 7/04, A43B 13/38

(54) **Elektrische Schuheinlagensohle mit Temperiereinrichtung**

(30) Priorität: 09.08.2013 DE 102013013215
(71) Anmelder: Marks, Peter, 65468 Trebur (DE); Rahman, Sanny, 64572 Büttelborn (DE)
(72) Erfinder: Marks, Peter, 65468 Trebur (DE); Rahman, Sanny, 64572 Büttelborn (DE)
(74) Vertreter: Behrens, Helmut

(57) **Zusammenfassung**

Die Erfindung betrifft eine elektrische Schuheinlagensohle (2), die eine Temperiereinrichtung (3) enthält, die mit einer wiederaufladbaren Batterie (4) und einer Steuereinrichtung (5) verbunden ist. Dabei ist die Erfindung dadurch gekennzeichnet, dass die Temperiereinrichtung (3), die wiederaufladbare Batterie (4) und die Steuereinrichtung (5) in der Schuheinlagensohle (2) integriert sind. Die Steuereinrichtung (5) ist dabei so ausgebildet, dass diese über eine kurze Funkverbindung (6) mit einem mitgeführten internetfähigen (Smartphone) Handy steuerbar ist.

## Beschreibung

Die Erfindung betrifft eine elektrische Schuheinlagensohle mit Temperiereinrichtung nach dem Oberbegriff des Patentanspruchs 1.

Zur Verbesserung des Tragekomforts von Schuhen sind herausnehmbare Schuheinlagensohlen und dergleichen bekannt, die zur Temperierung meist als Fußwärmer ausgebildet sind.

Ein derartiger Fußwärmer ist aus der DE 20 2006 007 638 U1 bekannt, der sich rund um den Fuß legt und an der Ferse geschlossen ist. Dabei ist zwischen dem Innenmaterial und einem Außenmaterial eine Schicht einer Leinsamen-Lavendel-Mischung angeordnet. Zur Temperierung des Fußwärmers wird dieser ein bis zwei Minuten in die Mikrowelle gelegt und danach wie eine Socke übergezogen. Dadurch wird der komplette Fuß wohltuend erwärmt. Allerdings kann die Wärmeabgabe nicht reguliert und der Fußwärmer nicht in herkömmlichen Schuhen verwendet werden.

Aus der DE 1 940 994 U1 ist ein elektrisch beheizbarer Fußwärmer bekannt, der in einer Schuheinlagensohle angeordnet ist, die in herkömmlichen Schuhen eingelegt werden kann. Dazu ist in der Schuheinlagensohle aus Asbest ein elektrischer Heizwiderstand mit Thermostat eingelegt, die mit einem Woll- oder Baumwollbezug umgeben ist. Zur elektrischen Speisung ist ein Tragegurt vorgesehen, in dem die Batterien angeordnet sind, den die Person um die Taille trägt. Dabei ist zur Einschaltung und zur Steuerung der Stromzufuhr am Tragegurt noch ein gut erreichbarer Stufenschalter angebracht, der über zwei in den Hosenbeinen geführte Kabel mit der jeweiligen Einlegesohle im Schuh verbunden ist. Allerdings ist ein derartiger elektrischer Fußwärmer zur Temperierung der Füße mit verkabeltem Tragegurt umständlich zu handhaben, und daher für einen kurzfristigen Einsatz meist zu aufwendig.

Eine weitere elektrische Fußsohlenheizung ist aus der DE 37 42 289 A1 bekannt, die aus einer losen Schuheinlagensohle mit Heizdrähten besteht, die über Drähte mit einem am erwärmten Körper getragenen wiederaufladbaren Batteriesatz verfügt. Dabei besitzt die Fußsohlenheizung eine elektronische Intervallsteuerung, um die Betriebsdauer zu erhöhen und die Temperatur auf einen angenehmen Wert einzustellen. Da der Batteriesatz am erwärmten Körper getragen werden muss und dieser auch mit den in den Schuhen eingelegten Einlagensohlen zu verdrahten ist, ist die Handhabung für einen häufigeren Gebrauch zu aufwendig.

Der Erfindung liegt deshalb die Aufgabe zugrunde, eine elektrische Schuheinlagensohle zur Temperierung der Füße so zu verbessern, dass sie in herkömmlichen Schuhen lose einlegbar und bequem zu handhaben ist.

Diese Aufgabe wird durch die im Patentanspruch 1 angegebene Erfindung gelöst. Weiterbildung und vorteilhafte Ausführungsbeispiele der Erfindung sind in den Unteransprüchen angegeben.

Die Erfindung hat den Vorteil, dass durch die integrierte Temperiereinrichtung, die integrierte Batterie und die integrierte Steuereinrichtung in der elektrischen Schuheinlagensohle eine kompakte Schuheinlagensohle geschaffen wurde, die ohne zusätzliche Verkabelung in herkömmlichen Schuhen zur Fußerwärmung oder Fußabkühlung wie herkömmliche Einlagensohlen verwendbar ist.

Die Erfindung hat weiterhin den Vorteil, dass die elektrische Schuheinlagensohle durch die integrierte Steuereinrichtung über eine kurze Funkverbindung mittels einer Bluetooth-Schnittstelle mit einem mitgeführten Smartphone verbindbar ist, durch das die Schuheinlagensohle jederzeit ein- oder ausschaltbar und in ihrer Heiz- oder Kühlleistung jederzeit individuell steuerbar ist.

Dabei hat eine besondere Ausführung der Erfindung durch den Einsatz von Peltier-Elementen den Vorteil, dass damit die Füße sowohl im Winter erwärmt als auch im Sommer gekühlt werden können. Der Einsatz derartiger Peltier-Elemente hat weiterhin den Vorteil, dass diese sehr flach und in Form einer Fußaufstandsfläche ausführbar sind und somit als flache integrierte Einlegeschicht in einer Schuheinlagensohle hergestellt werden können.

Eine weitere besondere Ausführung der Erfindung mit einem Einsatz einer wiederaufladbaren Batterie mit Lithium-Ionen-Zellen hat den Vorteil, dass diese in einem verhältnismäßig geringen Batterievolumen eine verhältnismäßig große Energiemenge speichern können und diese auch als flache integrierbare Einlageschicht für eine Schuheinlagensohle herstellbar sind. Dabei hat insbesondere die Kombination eines flachen Peltiermoduls mit einer flachen wiederaufladbaren Batterie aus Lithium-Ionen-Zellen den Vorteil, dass diese Einlageschichten in einer integrierfähigen Gesamthöhe ausführbar sind, die auch in herkömmlichen Schuhen eingelegt werden können.

Eine weitere besondere Ausführungsform der Erfindung mit einer zusätzlichen Einlageschicht mit einem integrierten flachen ebenen Sekundärteil einer Ladeschaltung in der Schuheinlagensohle hat den Vorteil, dass damit die integrierte wiederaufladbare Batterie auf einfache Weise nach jedem Gebrauch berührungslos wiederaufladbar ist. Dies hat zusätzlich den Vorteil, dass durch die Integration des Sekundärteils in der Schuheinlagensohle und ein einfaches Aufsetzen der Schuhe auf ein separates Ladegerät mit einem Primärteil einer Ladeschaltung auf einfache Weise die Batterien selbsttätig aufladbar sind ohne die Einlagensohlen aus den Schuhen herauszunehmen oder aufwendig über Kabelverbindungen galvanisch an das Ladegerät anzuschließen.

Die Erfindung wird anhand eines Ausführungsbeispiels, das in der Zeichnung dargestellt ist, näher erläutert. Es zeigen:
- Fig. 1:: eine elektrische Schuheinlagensohle mit integriertem Peltiermodul, einer Handysteuerung und einem Ladegerät, und
- Fig. 2:: ein Schaltbild eines induktiven Batterieladegerätes.

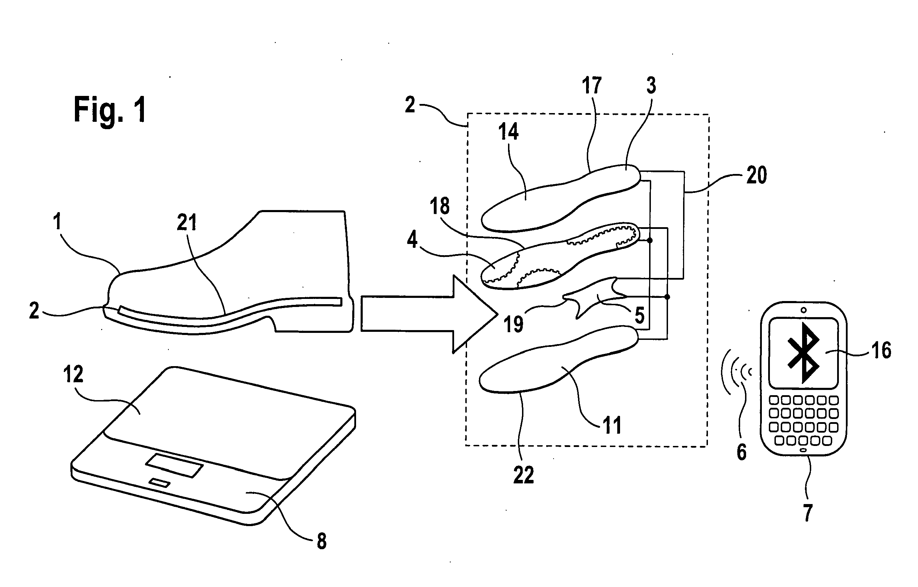

In Figur 1 der Zeichnung ist eine in einem Schuh 1 dargestellte elektrische Schuheinlagensohle 2 mit einem integrierten Peltiermodul 3 als Temperiereinrichtung, eine wiederaufladbare Batterie 4, eine elektronische Steuerschaltung 5 und ein Sekundärteil 11 einer Aufladeschaltung 9 ersichtlich, wobei die elektronische Steuerschaltung 5 über eine Funkverbindung 6 mit einem internetfähigen Smartphone 7 einschalt- und steuerbar ist, und die Batterie 4 über ein separates induktives Batterieladegerät 8 aufladbar ist.

Die elektrische Schuheinlagensohle 2 ist lose in einen herkömmlichen Schuh 1 einlegbar, wie dies auch bei anderen Schuheinlagen der Fall ist. Die elektrische Schuheinlagensohle 2 ist dabei im Querschnitt einer Fußaufstandsfläche 14 ausgebildet und als ca. 5 - 8 mm hohe flexible Sohle aus vier verschiedenen Schichten aufgebaut. Dabei besteht die Schuheinlagensohle 2 oben aus einer Temperiereinrichtung 3, die als obere Einlageschicht 17 ausgebildet ist und eine gleichmäßige Dicke oder Höhe von ca. 2 - 4 mm aufweist. Die Temperiereinrichtung 3 ist vorzugsweise als Peltiermodul ausgebildet, das sowohl zur Erwärmung als auch zur Kühlung schaltbar ist und ein sogenanntes Peltier-Element darstellt. Derartige Peltier-Elemente 3 stellen elektrothermische Wandler dar, die auf dem sogenannten Peltier-Effekt basieren und bei einem Stromdurchfluss eine Temperaturdifferenz erzeugen.

Diese Peltier-Elemente 3 sind als Handelsware erhältlich und bestehen aus zwei verschiedenen p- und n-dotierten Halbleiterquadern (z.B. Bismut-Tellurid, Silicium-Germanium), die abwechselnd oben und unten durch Metallbrücken miteinander verbunden sind. Dabei bilden die Metallbrücken zugleich die thermischen Kontaktflächen und sind zusätzlich durch eine aufliegende Folie 21 oder ein anderes Auflagematerial als Schutzschicht isoliert. Zum Stromfluss sind immer zwei unterschiedliche Quader so miteinander verbunden, dass sie eine Reihenschaltung bilden. Abhängig von der Stromstärke und -richtung kühlen sich oberen Verbindungsstellen ab, während sich die unteren erwärmen. Durch die Umkehrung der Stromrichtung ist mit derartigen Peltier-Elementen sowohl Kühlen als auch Heizen möglich. Sollten hingegen mit der Temperiereinrichtung 3 die Füße nur erwärmt werden, so könnte in die obere Einlegeschicht 17 auch ein elektrischer Heizdraht eingelegt werden.

Zur Erzeugung eines Wärme- oder Kühleffektes ist in jedem Fall die Zuführung eines elektrischen Stromes notwendig. Deshalb ist unter der oberen Einlegeschicht 17 eine mittlere Einlageschicht 18 mit der gleichen Fußaufstandsfläche 14 angeordnet, in die Zellen einer aufladbaren Batterie 4 in Form von Lithium-Ionen-Zellen angeordnet sind. Derartige Lithium-Ionen-Zellen sind auch in Flachbauweise herstellbar, so dass diese als flache, ca. 2 bis 3 mm hohe Einlageschicht 18 in der Schuheinlagensohle 2 integriert angeordnet werden.

Zur Inbetriebnahme und zur Einstellung der Heiz- oder Kühlleistung ist zusätzlich in der elektrischen Schuheinlagensohle 2 noch eine elektronische Steuerschaltung 5 integriert, die aus einer folienartigen Leiterplatine in Form einer kleineren Zwischenschicht 19 besteht auf der miniaturisierte elektronische Schaltelemente in SMD-Bauweise (SMD = Surface-mounted device) angeordnet sind, um die elektronischen Schaltvorgänge auszuführen. Dabei ist die Steuerschaltung 5 als digitale Schaltung ausgeführt, die auch programmgesteuerte Schaltelemente (Mikroprozessoren) enthält. Zur Ausführung der Steuerbefehle wird die Steuerschaltung 5 über eine kurze Funkverbindung 6 mit einem mitgeführten Handy 7 verbunden. Hierzu ist ein internetfähiges Handy 7 (Smartphone) notwendig, das mit einer speziellen Anwendersoftware (App) ausgestattet werden kann, durch das die elektronische Steuerschaltung 5 steuerbar ist. Dazu enthält die Steuerschaltung 5 eine sogenannte Bluetooth-Schnittstelle mit Treiber, um mit dem Handy über eine kurze Funkverbindung 6 (WPAN = Wireless Personal Local Area Network) kommunizieren zu können. Dabei wird die Kommunikation vorzugsweise nach dem Industriestandart IEEE-802.15.1 zur Datenübertragung zwischen den Geräten über kurze Distanzen per Funktechnik (WPAN) abgewickelt.

Über die Anwendungssoftware (App) ist dabei die Verbindung mit der Steuerschaltung 5 im Schuh 1 berührungslos herstellbar. Dazu ist das Anwendungsprogramm (App) so ausgebildet, dass damit die wiederaufladbare Batterie 4 an die Temperiereinrichtung 3 anschaltbar ist, so dass ein Stromfluss erfolgt, durch den eine Heizwirkung oder durch Umkehrung des Stromflusses eine Kühlwirkung eingestellt wird. Dazu ist gleichzeitig der Stromfluss entsprechend einer Lautstärkesteuerung durch + oder - Eingabe nahezu stufenlos einstellbar. In der Schuheinlagensohle 2 könnte vorzugsweise auch ein digitaler Temperaturaufnehmer integriert werden, so dass der Anwender über das Handy auch eine bestimmte Temperatur vorgeben könnte, die durch die Steuerschaltung 5 einstellbar ist. Dabei kann man über die Anwendersoftware (App) auch jederzeit die Einstellung verändern oder die Temperiereinrichtung 3 ausschalten. Zur Feststellung der noch vorhandenen Batterieladung könnte in der Steuerschaltung 5 auch eine Spannungsmessung integriert sein, mit der die Zellenspannung der Lithium-Ionen-Zellen von 3,6 V überwacht wird und bei abfallender Zellenspannung der entsprechende Ladezustand auf dem Display 16 des Handys 7 z.B. als Strichmuster angezeigt wird.

Diese 3 Einlageschichten 17, 18, 19 der Schuheinlagensohle 2 aus dem Peltiermodul 3, der wiederaufladbaren Batterie 4 als auch der elektronischen Steuerschaltung 5 sind über Kontaktelemente oder elektrische Leitungen 20 miteinander verbunden und so integriert, dass sie eine flache Schuheinlagensohle 2 miteinander bilden, die ohne zusätzliche Verkabelung lose in einem herkömmlichen Schuh 1 einlegbar ist. Dabei werden diese drei Einlageschichten 17, 18, 19 vorzugsweise mit einem Stoff oder einer thermisch gut leitbaren Folie 21 umgeben und sind insgesamt plastisch an die Auflagefläche der Schuhsole angepasst. Die drei Einlageschichten 17, 18, 19 können auch lose aufeinanderliegen und durch elektrische Steckkontakte miteinander zu einer elektrischen Schuheinlagensohle 2 verbunden sein.

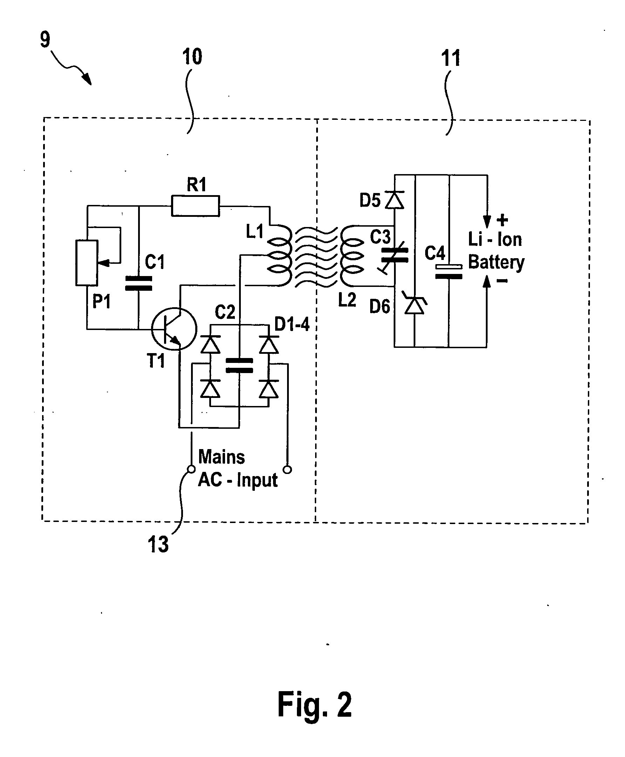

Zur automatischen Aufladung der aufladbaren Batteriezelle 4 ist zusätzlich ein induktives Ladegerät 8 vorgesehen, auf das nach dem Gebrauch vorzugsweise zwei Schuhe 1 aufgestellt werden können. Dazu ist eine zusätzliche Aufladeschaltung 9 sowohl im Ladegerät 8 als auch in der Schuheinlagensohle 2 vorgesehen, die in Fig. 2 der Zeichnung näher dargestellt ist. Die Aufladeschaltung 9 besteht dabei aus einem Primärteil 10 der im Ladegerät 8 und einem Sekundärteil 11 der in der Schuheinlagensohle 2 integriert ist. Dazu ist in der elektrischen Schuheinlagensohle 2 zusätzlich noch eine untere Einlageschicht 22 mit dem sekundären Teil 11 der Aufladeschaltung 9 vorgesehen, die ebenfalls aus einer folienartigen Schaltplatine besteht, auf der die elektronischen Schaltelemente vorzugsweise in SMD-Bauweise angeordnet sind. Dabei besteht der sekundäre Teil 11 der Aufladeschaltung 9 aus einer Induktionsspule L2, den Gleichrichtern D5, D6 und den Kondensatoren C3, C4, die an den Ausgangspolen +/- mit den Lithium-Ionen-Zellen 4 der mittleren Einlageschicht 18 verbunden sind.

Primärseitig befindet sich die Aufladeschaltung 9 in einem flachen Batterieladegerät 8 auf dessen oberer Abdeckplatte 12 die Schuhe 1 zum Aufladen aufstellbar sind. Unter der Abdeckplatte 12 befindet sich der primäre Teil 10 der Aufladeschaltung 9, der aus einer Gleichrichterschaltung D1 bis D4 mit Kondensator C2, einem Transistor T1, einem Potentiometerwiderstand P1, einem Kondesator C1, einem Widerstand R1 und einer Induktionsspule L1 besteht. Das Baterieladegerät 8 wird an seinen Eingangsklemmen 13 mit einer Netzspannung von vorzugsweise 220V gespeist. Dabei wird eine Wechselspannung von der primären Induktionsspule L1 in die sekundäre Induktionsspule L2 des sekundären Teils 11 der Aufladeschaltung 9 mit einer Ladespannung von ca. 4,3 V in die Einlagensohle 2 im Schuh 1 übertragen. Dadurch werden die wiederaufladbaren Batteriezellen 4, der auf der Abdeckplatte 12 abgestellten Schuhe 1 selbsttätig geladen, ohne dass dazu eine galvanische Anschaltung notwendig wäre. Bei der Ladeschaltung 9 handelt es sich um eine herkömmliche Ladeschaltung, wie sie bei induktiven Netzgeräten für die Ladung von Lithium-Ionen-Akkus bekannt sind. Zur Aufladung der Lithium-Ionen-Zellen 4 in der Schuheinlagensohle 2 sind aber auch andere Aufladeschaltungen einsetzbar, durch die eine Ladespannung in die Schuheinlagensohle 2 induktiv übertragbar ist. Allerdings könnte jede Schuheinlagensohle 2 auch über elektrische Kontaktstellen verfügen, durch die die Lithium-Ionen-Zellen 4 galvanisch mit einer Ladespannung verbunden werden könnten.

## Patentansprüche

1. Elektrische Schuheinlagensohle (2), die eine Temperiereinrichtung (3) enthält, die mit einer Batterie (4) und einer Steuereinrichtung (5) verbunden ist, **dadurch gekennzeichnet, dass** die Temperiereinrichtung (3) die Batterie (4) und die Steuereinrichtung (5) in der Schuheinlagensohle (2) integriert sind, wobei die Steuereinrichtung (5) so ausgebildet ist, dass diese über eine Funkverbindung (6) mit einem internetfähigen (Smartphone) Handy steuerbar ist.

2. Elektrische Schuheinlagensohle (2) nach Anspruch 1, **dadurch gekennzeichnet, dass** die Temperiereinrichtung (3), die Batterie (4) und die Steuereinrichtung (5) jeweils als eine flache, ebene obere Einlageschicht (17), eine mittlere Einlageschicht (18) und eine Zwischenschicht (19) ausgebildet ist, deren Querschnittsfläche jeweils maximal der Fußaufstandsfläche (14) entspricht und die vertikal übereinander angeordnet und die mechanisch zu einer Einheit miteinander verbunden sind.

3. Elektrische Schuheinlagensohle (2) nach Anspruch 1 oder 2, **dadurch gekennzeichnet, dass** die Temperiereinrichtung (3) aus einem oder mehreren Peltier-Elementen besteht, die zu einem flachen ebenen Peltiermodul (3) ausgebildet sind, dessen Querschnittsfläche höchstens der Fußaufstandsfläche (14) und dessen Höhe oder Dicke höchstens 8 mm beträgt.

4. Elektrische Schuheinlagensohle (2) nach Anspruch 3, **dadurch gekennzeichnet, dass** das Peltiermodul (3) mindestens auf seinen beiden Querschnittsflächen mit einer elektrisch isolierenden Folie (21) oder einer Schutzschicht abgedeckt ist.

5. Elektrische Schuheinlagensohle (2) nach Anspruch 1 oder 2, **dadurch gekennzeichnet, dass** die Temperiereinrichtung (3) aus einer hitzebeständigen Einlageschicht (17) besteht, in der ein oder mehrere elektrische Heizdrähte eingelegt sind.

6. Elektrische Schuheinlagensohle (2) nach einem der vorhergehenden Ansprüche, **dadurch gekennzeichnet, dass** die Batterie (4) wiederaufladbar ist und eine oder mehrere Lithium-Ionen-Zellen enthält.

7. Elektrische Schuheinlagensohle (2) nach einem der vorhergehenden Ansprüche, **dadurch gekennzeichnet, dass** die Steuereinrichtung (5) eine digitale elektronische Steuerschaltung enthält, die mit einem internetfähigen (Smartphone) Handy über eine sogenannte Bluetooth-Schnittstelle berührungslos verbindbar und so ausgebildet ist, dass die Schuheinlagensohle (2) über eine spezielle Anwendersoftware (App) auf dem Handy (7) ein- und ausschaltbar und in der Heiz- und/oder Kühlleistung individuell steuerbar ist.

8. Elektrische Schuheinlagensohle (2) nach einem der vorhergehenden Ansprüche, **dadurch gekennzeichnet, dass** die Schuheinlagensohle (2) eine zusätzliche Einlageschicht (22) enthält, in der ein sekundärer Teil (11) einer Ladeschaltung (9) zur Batterieaufladung angeordnet ist, die induktiv mit einem separaten Primärteil (10) der Ladeschaltung (9) in einem separaten Batterieladegerät (8) verbindbar ist.
